# EUROPÄISCHE PATENTSCHRIFT

(11) **EP 0 842 651 B1**
(45) Veröffentlichungstag und Bekanntmachung des Hinweises auf die Patenterteilung: **12.03.2003**
(21) Anmeldenummer: 97117983.3
(22) Anmeldetag: 16.10.1997
(51) Int. Cl.: A61K 6/00

(54) **Primer zur Vorbereitung einer Zahnkavität für eine Kompositfüllung**
Primer for preparing a tooth cavity before the application of a composite filler
Couche primaire pour la préparation d'une cavité dentaire avant l'application d'une obturation en matériau composite

(30) Priorität: 14.11.1996 DE 19647140; 09.12.1996 DE 19651121
(43) Veröffentlichungstag der Anmeldung: 20.05.1998
(73) Patentinhaber: Ernst Mühlbauer GmbH & Co.KG, 25870 Norderfriedrichskoog (DE)
(72) Erfinder: Reinhardt, Klaus-Jürgen, Prof. Dr., 48161 Münster (DE); Lück, Rainer, Dr. SC, 25436 Tornesch (DE)
(74) Vertreter: Glawe, Delfs, Moll & Partner

(56) Entgegenhaltungen:
- EP-A- 0 161 337
- EP-A- 0 661 034
- US-A- 5 204 383

## Beschreibung

Gegenstand der Erfindung ist die Verwendung einer bestimmten Mischung zur Herstellung eines Mittels zur Vorbereitung einer Zahnfläche zur Verbindung mit einem dentalen Kompositmaterials sowie ein entsprechend hergestelltes Mittel.

Vor dem Füllen von Zahnkavitäten mit einem polymerisierbaren Kompositmaterial auf Kunststoffbasis muß die Zahnsubstanz (Dentin oder Schmelz) vorbehandelt werden, um eine gute Haftung des Komposits daran sicherzustellen. Ein guter Verbund zwischen Zahn und Füllung ist wichtig, da alle bekannten Kompositmaterialien insbesondere in der Anfangsphase der Polymerisation schrumpfen. Bei mangelnder Haftung kommt es zu einer Randspaltbildung, in den Randspalt können Bakterien eindringen und Sekundärkaries hervorrufen und/oder die Pulpa schädigen.

Aus offenkundiger Vorbenutzung ist es bekannt, diese Vorbehandlung vor dem Legen der Füllung in vier Schritten auszuführen. Zunächst wird der Zahnschmelz mit einer sauren Ätzlösung, die in der Regel Phosphorsäure enthält, angeätzt. Zum Ätzen von Dentin muß ggf. eine andere Ätzflüssigkeit benutzt werden. Im nächsten Schritt wird die Ätzlösung abgewaschen. Im dritten Schritt wird ein Primer auf die geätzte Fläche aufgetragen und (ggf. durch Licht) ausgehärtet. Primer enthalten in der Regel polymerisierbare Säuren und sind beispielsweise in DE-A 35 36 077, US-A 4 514 342, US-A 4 388 421 und DE-A 40 32 882 beschrieben. In einem vierten Schritt wird auf den ausgehärteten Primer ein sog. Bond aufgetragen, der in der Regel ebenfalls mit Licht ausgehärtet werden muß. Als Bondingsysteme werden in der Regel Methacrylate, insbesondere hydrophile Methacrylate (beispielsweise Hydroxyethylmethacrylat) verwendet. In der Regel enthalten Bondingsysteme zusätzlich einen geringen Anteil von Säuren, insbesondere polymerisierbare Säuren. Der Bond soll dem anschließend zu legenden Kompositmaterial eine Grundlage bieten, in die dieser einpolymerisieren kann.

Aus EP-A-0 161 337 ist ein photopolymerisierbarer phosphathaltiger dentaler Haftvermittlerlack bekannt, bei dem ein Methacryloyloxyethylphosphat mit einem Photopolymerisationskatalysator in Aceton gelöst ist. Als Photopolymerisationskatalysator kann Campherchinoh und ein Amin verwendet werden.

Aufgabe der Erfindung ist es, ein Mittel zur Vorbereitung einer Zahnfläche wie beispielsweise einer Zahnkavität zur Verbindung mit einem dentalen Kompositmaterial bereitzustellen, das einfacher und mit weniger Zeitaufwand anzuwenden ist und dennoch einen guten Verbund zwischen der Zahnsubstanz und der Kompositfüllung gewährleistet.

Erfindungsgemäß wird zur Herstellung eines solchen Mittels eine Mischung verwendet, die enthält:
a) 1 - 5 Gew.-% Phosphorsäure;
b) 10 - 90 Gew.-% Phosphorsäuremono- und/oder -diester der Formel 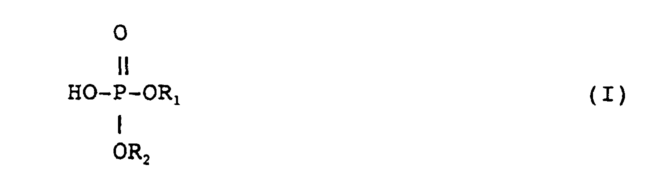 wobei
   R₁ gleich 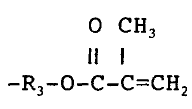 ist;
   R₂ gleich H oder 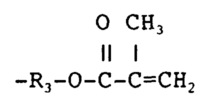 ist;
   R₃ ausgewählt ist aus der Gruppe bestehend aus Alkylengruppen, Alkylenoxygruppen mit einer oder mehreren Alkylenoxyeinheiten, mit einer oder mehreren Hydroxygruppen substituierten Alkylen- oder Alkylenoxygruppen;
c) 1 - 15 Gew.-% Phosphorsäuretriester der Formel

   O=P(OR₁)₃ (II)

   mit der oben angegebenen Bedeutung für R₁;
d) 3 - 50 Gew.-% Diphosphate der Formel 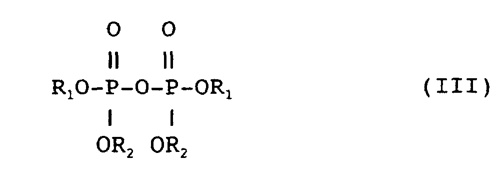 mit den oben angegebenen Bedeutungen für R₁ und R₂, wobei R₂ in Formel (III) gleich oder verschieden sein kann.

Die Erfindung ermöglicht es, eine Zahnfläche in einem einzigen Schritt zum Legen einer Kompositfüllung vorzubereiten. Das erfindungsgemäße Mittel enthält eine Mischung aus Phosphorsäure, einem Hauptanteil Phosphorsäuremono- und/oder -diestern, ferner Phosphorsäuretriester und Diphosphate. Der geringe Phosphorsäureanteil dient dem vorbereitenden Ätzen der Zahnfläche. Die erfindungsgemäß verwendeten Phosphorsäureester sind Moleküle, deren saure Hydroxygruppen sich chemisch mit dem Kalzium der Zahnoberfläche verbinden können. Die teilveresterte Phosphorsäuregruppe ist über eine als Spacer dienende Alkylen- oder Alkylenoxygruppe (die ggf. Hydroxygruppen trägt) mit einer Methacrylatgruppe verbunden. Die Methacrylatgruppen können teilweise polymerisieren und so einen Film auf der Zahnoberfläche bilden, in die das Kompositmaterial einpolymerisieren kann. Die Phosphorsäureenden der Moleküle können aufgrund ihrer guten Beweglichkeit in Dentintubuli eindringen und so die mechanische Adhäsion an der Zahnfläche verbessern.

Die Prozentangaben in Anspruch 1 beziehen sich auf den sog. Harzanteil der Mischung, der bei einem lösemittelfreien Gemisch 100 % beträgt. Bei Zusatz eines Lösemittels verringert er sich entsprechend der zugesetzte Lösemittelmenge.

Über Spacer mit Methacrylatgruppen verknüpfte Phosphorsäureester sind bisher als kombiniertes Ätzmittel und Primer sowie ggf. Haftmittel (Bond) für Zahnoberflächen nicht in Betracht gezogen worden. Ein Grund dafür liegt darin, daß solche Phosphorsäurederivate in reiner Form nicht stabil und somit nicht lagerfähig sind. Die Haftkraft solcher reiner Phosphorsäurederivate ist gering, wie unten anhand eines Vergleichsbeispiels noch erläutert wird.

Überraschenderweise hat sich gezeigt, daß die erfindungsgemäße Mischung von Phosphorsäure, -mono-, di- und -triestern sowie Diphosphaten sowohl über eine gute Lagerstabilität als auch eine sehr hohe Haftkraft verfügt. Ätzen, Primen und ggf. Bonding lassen sich mit dieser Mischung in einem einzigen Verfahrensschritt durchführen.

Im Rahmen der Erfindung umfaßt der in Anspruch 1 verwendete Begriff "Vorbereitung einer Zahnfläche zur Verbindung mit einem dentalen Kompositmaterial" sowohl das Ätzen der Zahnfläche als auch das Primen. Das Primen beinhaltet die Herstellung einer guten Verbindung zwischen Primer und Zahnoberfläche, u.a. durch Eindringen der an den Spacerabschnitten der Moleküle angeordneten Phosphorsäureenden in die Dentintubuli der Zahnsubstanz. Der Primer bewirkt eine gute mechanische Adhäsion an der Zahnfläche. Die im Stand der Technik getrennt ausgeführten Schritte des Ätzens und Primens können erfindungsgemäß in einem einzigen Schritt ausgeführt werden. Vorzugsweise umfaßt der genannte Begriff zusätzlich auch das sogenannte Bonding. Mit Bonding bezeichnet man das Schaffen einer guten Haftgrundlage für das anschließende Einpolymerisieren des Kompositmaterials. Im Stand der Technik verwendete Primer bieten häufig eine nicht ausreichende Grundlage für dieses Einpolymerisieren, so daß zusätzlich ein Bond zum Erstellen einer ausreichenden mechanischen Adhäsion mit dem Komposit aufgetragen werden muß. Erfindungsgemäß ist dies vorzugsweise nicht erforderlich, die verwendete Mischung macht ein zusätzliches Bonding überflüssig und bildet selbst eine gute Haftgrundlage für das Kompositmaterial.

Die Spacergruppe in den Phosphorsäurederivaten wird vorzugsweise ausgewählt aus der Gruppe bestehend aus Alkylengruppen mit 2 - 4 C-Atomen, Ethylenoxy- oder Propylenoxygruppen, die aus bis zu 4 Glykoleinheiten (Ethylen- oder Propylenglykoleinheiten) bestehen können, sowie Alkylengruppen mit 2 - 4 C-Atomen, die mit einer oder zwei Hydroxygruppen substituiert sind.

Bevorzugt als Spacer sind Ethylen- oder Propylengruppen, ferner eine Gruppe der Formel 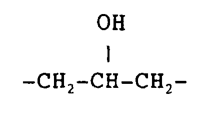 oder ein Isomeres davon.

In der erfindungsgemäßen Mischung besteht der Hauptanteil aus Phosphorsäuremono- und -diestern, er macht vorzugsweise 30 - 90 Gew.-%, weiter vorzugsweise 40 - 80 Gew.-% bezogen auf den Harzanteil aus. Weiter bevorzugt ist ein Verhältnis von 20 - 35 Gew.-% Phosphorsäuremonoester und 25 - 45 Gew.-% Phosphorsäurediester. Der Phosphorsäuregehalt der Mischung beträgt vorzugsweise 1 - 3 Gew.-%.

Die erfindungsgemäße Mischung kann direkt auf die zu behandelnde Zahnoberfläche aufgetragen werden, es kann jedoch zusätzlich ein Lösungsmittel hinzugefügt werden. Geeignete Lösungsmittel sind Aceton, Ethanol, Isopropanol, THF und Wasser. Aceton, Ethanol und Wasser sind bevorzugte Lösungsmittel. Der Anteil des Lösungsmittels oder der Lösungsmittel an der Gesamtmischung beträgt vorzugsweise 30 - 95 Gew.-%.

Es ist möglich, die erfindungsgemäßen Mischungen ohne einen hinzugefügten Polymerisationsinitiator zu verwenden. Die Polymerisation wird in diesem Fall nach Legen der Füllung ausgehend vom Kompositmaterial gestartet. Es kann jedoch ein Polymerisationsinitiator, bevorzugt ein Photostarter, hinzugefügt werden. Photostarter sind dem Fachmann geläufig und beispielsweise in FR-A 2 156 760 und GB-A 1 408 265 beschrieben. Es kann sich dabei beispielsweise um aromatische Ketone oder um eine Mischung aus Diketonen und tertiären Aminen handeln. Campherchinon, ggf. in Verbindung mit einem tertiären Amin, ist als Photostarter bevorzugt, der durch sichtbares Licht initiiert wird. Ein durch UV-Licht initiierter Photostarter ist beispielsweise 1,2-Diphenyl-2,3-dimethoxymethanon.

Die Erfindung wird nachfolgend anhand eines Ausführungsbeispieles und eines Vergleichsbeispieles erläutert.

### Beispiel 1

25 g Phosphorsäuretrichlorid werden in 100 ml THF gelöst. 15 g HEMA werden unter Kühlen und Feuchtigkeitsausschluß portionsweise zugetropft. Die Mischung wird drei Tage gerührt, anschließend mit 2 g Wasser versetzt und weitere 24 h gerührt. Das Lösungsmittel und die entstandene HCl werden unter Vakuum entfernt.

Die Reaktionsmischung wird mittels ³¹P-NMR-Spektroskopie analysiert. Sie hat folgende Zusammensetzung:

2% Phosphorsäure, 25% Phosphorsäuremonoester, 35% Phosphorsäurediester, 7% Phosphorsäuretriester, 31% Diphosphat.

Sämtliche Prozentangaben sind Gewichtsprozente.

25 g dieser Reaktionsmischung werden in 75 g Aceton gelöst. Anschließend werden 0,1 g Campherchinon und 0,05 g Triethanolamin zugesetzt.

### Vergleichsbeispiel 1

25 g Bis(2-methacryloxyethyl)phosphat werden in 75 g Aceton gelöst und mit 0,1 g Campherchinon und 0,05 g Triethanolamin versetzt.

Die Haftwerte, die ein Kompositmaterial auf Rinderdentin unter Verwendung der Mischung des Beispiels 1 bzw. Vergleichsbeispiels 1 erreicht, werden nach dem im ISO-Entwurf TR 11405 beschriebenen Verfahren gemessen. Es ergeben sich folgende Werte:
- Beispiel 1:: 18 MPa
- Vergleichsbeispiel 1:: 12 MPa

## Patentansprüche

1. Verwendung einer Mischung, die enthält
a) 1 - 5 Gew.-% Phosphorsäure;
b) 10 - 90 Gew.-% Phosphorsäuremono- und/oder -diester der Formel 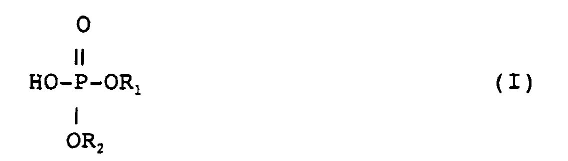 wobei
R₁ gleich 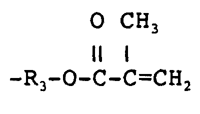 ist;
R₂ gleich H oder 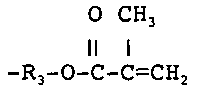 ist;
R₃ ausgewählt ist aus der Gruppe bestehend aus Alkylengruppen, Alkylenoxygruppen mit einer oder mehreren Alkylenoxyeinheiten, mit einer oder mehreren Hydroxygruppen substituierten Alkylen- oder Alkylenoxygruppen;
c) 1 - 15 Gew.-% Phosphorsäuretriester der Formel
O=P(OR₁)₃ (II)
mit der oben angegebenen Bedeutung für R₁;
d) 3 - 50 Gew.-% Diphosphate der Formel 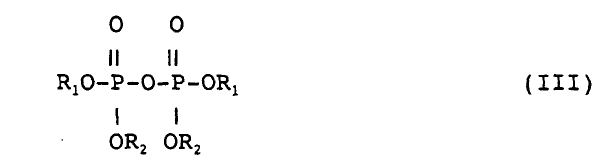 mit den oben angegebenen Bedeutungen für R₁ und R₂, wobei R₂ in Formel (III) gleich oder verschieden sein kann und wobei die Gew.-%-Angaben bezogen sind auf den lösemittelfreien Anteil der Mischung;
zur Herstellung eines Mittels zur Vorbereitung einer Zahnfläche zur Verbindung mit einem dentalen Kompositmaterial.

2. Verwendung nach Anspruch 1, **dadurch gekennzeichnet, daß** R₃ ausgewählt ist aus der Gruppe bestehend aus Alkylengruppen mit 2 - 4 C-Atomen, Ethylenoxy- oder Propylenoxygruppen aus bis zu 4 Glykoleinheiten, Alkylengruppen mit 2 - 4 C-Atomen, die mit einer oder zwei Hydroxygruppen substituiert sind.

3. Verwendung nach Anspruch 2, **dadurch gekennzeichnet, daß** R₃ eine Ethylen- oder Propylengruppe ist.

4. Verwendung nach Anspruch 2, **dadurch gekennzeichnet, daß** R₃ gleich 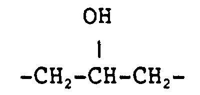 oder ein Isomeres davon ist.

5. Verwendung nach einem der Ansprüche 1 bis 4, **dadurch gekennzeichnet, daß** der Anteil der Phosphorsäuremonound/oder -diester an der Mischung 30 - 90 Gew.-%, vorzugsweise 40 - 80 Gew.-% beträgt.

6. Verwendung nach Anspruch 5, **dadurch gekennzeichnet, daß** die Mischung 20 - 35 Gew.-% Phosphorsäuremonoester und 25 - 45 Gew.-% Phosphorsäurediester enthält.

7. Verwendung nach einem der Ansprüche 1 bis 6, **dadurch gekennzeichnet, daß** die Mischung 1 - 3 Gew.-% Phosphorsäure enthält.

8. Verwendung nach einem der Ansprüche 1 bis 7, **dadurch gekennzeichnet, daß** zusätzlich ein Lösungsmittel hinzugefügt wird.

9. Verwendung nach Anspruch 8, **dadurch gekennzeichnet, daß** der Anteil des Lösungsmittels an der gesamten Mischung 30 - 95 Gew.-% beträgt.

10. Verwendung nach Anspruch 8 oder 9, **dadurch gekennzeichnet, daß** als Lösungsmittel Ethanol oder Aceton verwendet wird.

11. Verwendung nach Anspruch 8 oder 9, **dadurch gekennzeichnet, daß** als Lösungsmittel Wasser verwendet wird.

12. Verwendung nach einem der Ansprüche 1 bis 10, **dadurch gekennzeichnet, daß** der Mischung zusätzlich ein Photostarter hinzugefügt wird.

13. Mittel zum Vorbereiten einer Zahnfläche zur Verbindung mit einem dentalen Kompositmaterial, **dadurch gekennzeichnet, daß** es enthält:
a) 1 - 5 Gew.-% Phosphorsäure;
b) 10 - 90 Gew.-% Phosphorsäuremono- und/oder -diester der Formel 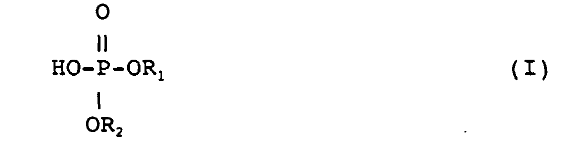 wobei
R₁ gleich 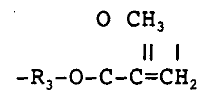 ist;
R₂ gleich H oder 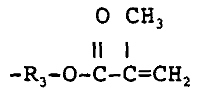 ist;
R₃ ausgewählt ist aus der Gruppe bestehend aus Alkylengruppen, Alkylenoxygruppen mit einer oder mehreren Alkylenoxyeinheiten, mit einer oder mehreren Hydroxygruppen substituierten Alkylen- oder Alkylenoxygruppen;
c) 1 - 15 Gew.-% Phosphorsäuretriester der Formel
O=P(OR₁)₃ (II)
mit der oben angegebenen Bedeutung für R₁;
d) 3 - 50 Gew.-% Diphosphate der Formel 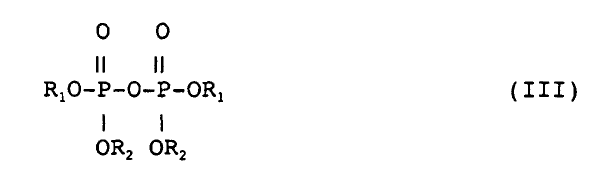 mit den oben angegebenen Bedeutungen für R₁ und R₂, wobei R₂ in Formel (III) gleich oder verschieden sein kann, wobei die Gew.-%-Angaben bezogen sind auf den lösemittelfreien Anteil der Mischung.

## Claims

1. Use of a mixture which comprises
a) 1 - 5% by weight of phosphoric acid;
b) 10 - 90% by weight of phosphoric acid mono- and/or diesters of the formula 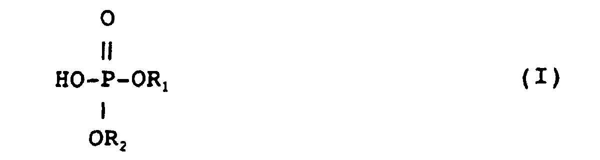 wherein
R₁ is 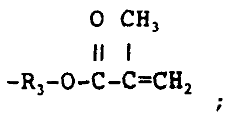 R₂ is H or 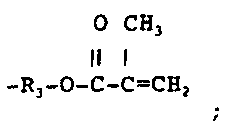 R₃ is chosen from the group consisting of alkylene groups, alkyleneoxy groups having one or more alkyleneoxy units and alkylene or alkyleneoxy groups substituted by one or more hydroxyl groups;
c) 1 - 15% by weight of phosphoric acid triesters of the formula
O=P(OR₁)₃ (II)
with the abovementioned meaning for R₁; and
d) 3 - 50% by weight of diphosphates of the formula 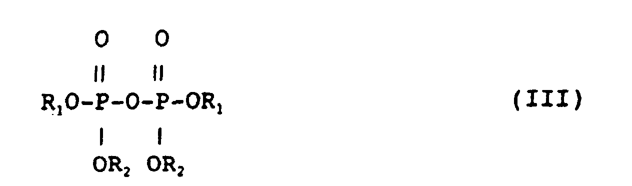 with the abovementioned meanings for R₁ and R₂, wherein R₂ in formula (III) can be identical or different and wherein the % by weight data are based on the solvent-free part of the mixture;
for the preparation of a composition for preparing a tooth surface for joining with a dental composite material.

2. , Use according to Claim 1, **characterized in that** R₃ is chosen from the group consisting of alkylene groups having 2-4 C atoms, ethyleneoxy or propyleneoxy groups of up to 4 glycol units, and alkylene groups having 2-4 C atoms, which are substituted by one or two hydroxyl groups.

3. Use according to Claim 2, **characterized in that** R₃ is an ethylene or propylene group.

4. Use according to Claim 2, **characterized in that** R₃ is 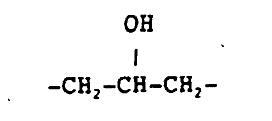 or an isomer thereof.

5. Use according to one of Claims 1 to 4, **characterized in that** the proportion of phosphoric acid mono- and/or diesters in the mixture is 30-90% by weight, preferably 40-80% by weight.

6. Use according to Claim 5, **characterized in that** the mixture comprises 20-35% by weight of phosphoric acid monoesters and 25-45% by weight of phosphoric acid diesters.

7. Use according to one of Claims 1 to 6, **characterized in that** the mixture comprises 1-3% by weight of phosphoric acid.

8. Use according to one of Claims 1 to 7, **characterized in that** a solvent is additionally added.

9. Use according to Claim 8, **characterized in that** the content of solvent in the total mixture is 30-95% by weight.

10. Use according to Claim 8 or 9, **characterized in that** ethanol or acetone is used as the solvent.

11. Use according to Claim 8 or 9, **characterized in that** water is used as the solvent.

12. Use according to one of Claims 1 to 10, **characterized in that** a photoinitiator is additionally added to the mixture.

13. Composition for preparing a tooth surface for joining with a dental composite material, **characterized in that** it comprises:
a) 1 - 5% by weight of phosphoric acid;
b) 10 - 90% by weight of phosphoric acid mono- and/or diesters of the formula 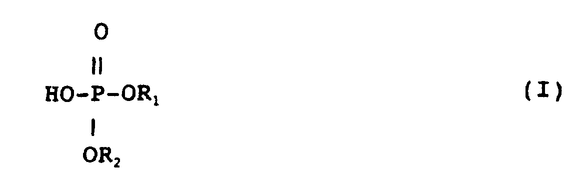 wherein
R₁ is 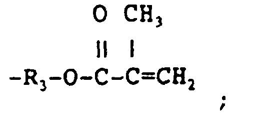 R₂ is H or 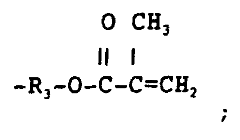 R₃ is chosen from the group consisting of alkylene groups, alkyleneoxy groups having one or more alkyleneoxy units and alkylene or alkyleneoxy groups substituted by one or more hydroxyl groups;
c) 1 - 15% by weight of phosphoric acid triesters of the formula
O=P(OR₁)₃ (II)
with the abovementioned meaning for R₁; and
d) 3 - 50% by weight of diphosphates of the formula 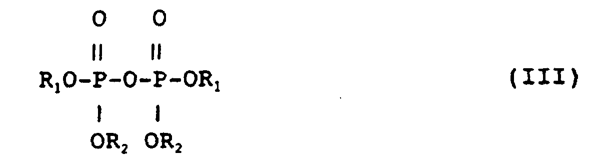 with the abovementioned meanings for R₁ and R₂, wherein R₂ in formula (III) can be identical or different, wherein the % by weight data are based on the solvent-free part of the mixture.

## Revendications

1. Utilisation d'un mélange contenant
a) 1 à 5 % en poids d'acide phosphorique ;
b) 10 à 90 % en poids de monoester et/ou de diester d'acide phosphorique de formule 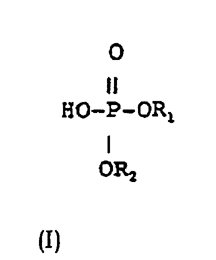 dans laquelle
R₁ est 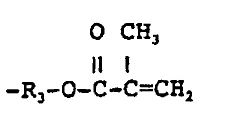 R₂ est H ou 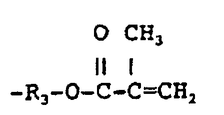 R₃ est choisi dans le groupe comprenant des groupes alkylène, des groupes alkylénoxy avec une ou plusieurs unités alkylénoxy, des groupes alkylène ou alkylénoxy substitués avec un ou plusieurs groupes hydroxy ;
c) 1 à 15 % en poids de triester d'acide phosphorique de formule
O=P(OR₁)₃ (II)
R₁ ayant la signification précisée ci-dessus ;
d) 3 à 50 % en poids de diphosphate de formule 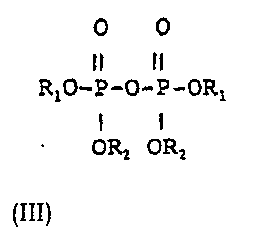 R₁ et R₂ ayant la signification précisée ci-dessus, R₂ dans la formule (III) pouvant être identique ou différent et les pourcentages en poids étant les pourcentages en poids de la part sans solvant du mélange ;
pour fabriquer un mélange pour la préparation d'une surface dentaire pour liaison avec un matériau composite dentaire.

2. Utilisation selon la revendication 1 **caractérisée en ce que** R₃ est choisi dans le groupe comprenant des groupes alkylène avec 2 à 4 atomes de carbone, des groupes éthylénoxy ou propylénoxy avec jusqu'à 4 unités glycol, des groupes alkylène avec 2 à 4 atomes de carbone substitués avec un ou deux groupes hydroxy.

3. Utilisation selon la revendication 2 **caractérisée en ce que** R₃ est un groupe éthylène ou propylène.

4. Utilisation selon la revendication 2 **caractérisée en ce que** R₁ est 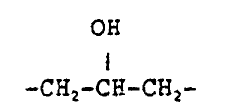 ou un de ses isomères.

5. Utilisation selon l'une des revendications 1 à 4 **caractérisée en ce que** la part de monoester et/ou de diester d'acide phosphorique dans le mélange s'élève à 30 à 90 % en poids, de préférence à 40 à 80 % en poids.

6. Utilisation selon la revendication 5 **caractérisée en ce que** le mélange contient 20 à 35 % en poids de monoester d'acide phosphorique et 25 à 45 % en poids de diester d'acide phosphorique.

7. Utilisation selon l'une des revendications 1 à 6 **caractérisée en ce que** le mélange contient 1 à 3 % en poids d'acide phosphorique.

8. Utilisation selon l'une des revendications 1 à 7 **caractérisée en ce qu'**en outre un solvant est ajouté.

9. Utilisation selon la revendication 8 **caractérisée en ce que** la part du solvant sur l'ensemble du mélange s'élève à 30 à 95 % en poids.

10. Utilisation selon la revendication 8 ou 9, **caractérisée en ce que** l'on utilise comme solvant de l'éthanol ou de l'acétone.

11. Utilisation selon la revendication 8 ou 9 **caractérisée en ce que** l'on utilise comme solvant de l'eau.

12. Utilisation selon l'une des revendications 1 à 10 **caractérisée en ce que** le mélange est en outre additionné d'un photo-initiateur.

13. Moyen de préparation d'une surface de dent pour liaison à un matériau composite dentaire **caractérisé en ce qu'**il contient :
a) 1 à 5 % en poids d'acide phosphorique ;
b) 10 à 90 % en poids de monoester et/ou de diester d'acide phosphorique de formule 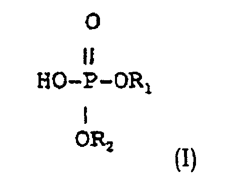 dans laquelle
R₁ est 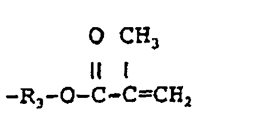 R₂ est H ou 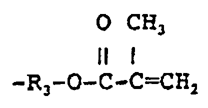 R₃ est choisi dans le groupe comprenant des groupes alkylène, des groupes alkylénoxy avec une ou plusieurs unités alkylénoxy, des groupes alkylène ou alkylénoxy substitués avec un ou plusieurs groupes hydroxy ;
c) 1 à 15 % en poids de triester d'acide phosphorique de formule
O=P(OR₁)₃ (II)
R₁ ayant la signification précisée ci-dessus ;
d) 3 à 50 % en poids de diphosphate de formule 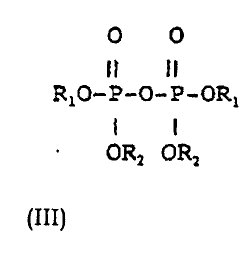 R₁ et R₂ ayant la signification précisée ci-dessus, R₂ dans la formule (III) pouvant être identique ou différent et les pourcentages en poids étant les pourcentages en poids de la part sans solvant du mélange.
